Europäisches Patentamt

European Patent Office

Office européen des brevets

(19)

(11) Veröffentlichungsnummer: **0 285 603**
**A2**

(12) **EUROPÄISCHE PATENTANMELDUNG**

(21) Anmeldenummer: 88890082.6

(22) Anmeldetag: 31.03.88

(51) Int. Cl.4: **G 01 N 33/68**

(30) Priorität: 03.04.87 AT 819/87

(43) Veröffentlichungstag der Anmeldung:
05.10.88 Patentblatt 88/40

(84) Benannte Vertragsstaaten:
BE CH DE ES FR GB IT LI LU NL SE

(71) Anmelder: BIOMEDICA HANDELSGESELLSCHAFT
M.B.H.
Rennweg 2
A-1030 Wien (AT)

(72) Erfinder: Woloszczuk, Wolfgang, Dr.
Endemanngasse 6-48/I/39
A-1230 Wien (AT)

(74) Vertreter: Casati, Wilhelm, Dipl.-Ing. et al
Patentanwälte Casati, Wilhelm, Dipl.-Ing. Itze, Peter,
Dipl.-Ing. Amerlingstrasse 8
A-1061 Wien (AT)

(54) Verfahren zur Bestimmung von Cyclosporin-A im Blut.

(57) Bei einem Verfahren zur Bestimmung des aktuellen Pegels
von Cyclosporin-A bzw. des diesem verwandter Immunsuppressiva sowie jenes deren biologisch aktiver Metaboliten im
Blut wird der zu untersuchenden Blutprobe eine definierte
Menge an Cyclophilin und eine definierte Menge an markierter
an Cyclophilin bindbarer Substanz zugesetzt, worauf dann
durch Ermittlung des an das Cyclophilin gebundenen Anteils an
markierter Substanz durch Vergleich mit Standards der im Blut
befindliche Pegel an Cyclosporin-A sowie dessen Metaboliten
bestimmt wird.

EP 0 285 603 A2

## Beschreibung

Verfahren zur bestimmung des aktuellen Pegels von Cyclosporin-A bzw. des diesem verwandter Immunsuppressiva sowie jenes deren biologisch aktiver metaboliten im Blut

Die Erfindung bezieht sich auf ein Verfahren zur Bestimmung des aktuellen Pegels von Cyclosporin-A bzw. des diesem verwandter Immunsuppressiva sowie jenes deren biologisch aktiver Metaboliten im Blut.

Cyclosporin-A wird seit Jahren erfolgreich zur Unterdrückung der immunologischen Abstoßungsreaktionen, z.B. nach Organtransplantationen, eingesetzt. Cyclosporin-A sowie eventuell dessen biologisch aktive Metaboliten sind dabei in der Lage, die gegen das Transplantat gerichteten Immunreaktionen weitgehend zu unterdrücken, wobei die übrigen Funktionen des Immunsystems nur in verhältnismäßig geringem Maße beeinträchtigt werden. Einen großen Nachteil der Cyclosporintherapie stellt allerdings die hohe Nephrotoxizität dieser Verbindung dar. Es ist daher unerläßlich, den aktuellen Pegel des Immunsuppressivums ständig zu kontrollieren um danach das Immunsuppressivum genau zu dosieren. Ist die Dosierung zu niedrig, kann es zu Abstoßungsreaktionen kommen, ist hingegen die Dosierung zu hoch, so können die Nieren schwer geschädigt werden.

Zur Messung des Pegels von Cyclosporin-A bzw. diesem verwandter Immunsuppressiva stehen derzeit zwei völlig unterschiedliche Methoden zur verfügung. Es handelt sich dabei einerseits um eine Radioimmunobestimmung (RIA) und anderseits um die Hochdruckflüssigkeitchromatographie (HPLC). Mit der HPLC-Methode wird ausschließlich das Immunsuppressivum selbst bestimmt, nicht jedoch die zahlreichen Metaboliten, die haupsächlich in der Leber gebildet werden und bis über 80% der Gesamtmenge an Immunsuppressivum betragen können. Sowohl über die immunsuppressive als auch über die nephrotoxische Wirkung dieser Metaboliten besteht jedoch wenig Klarheit. In vitro erhaltene Ergebnisse führen dabei häufig zu widersprüchlichen Aussagen. Im RIA-Rest wiederum wird nur ein Teil der Metaboliten erfaßt, nämlich jener, der Kreuzreaktionen zu dem gegen das Immunsuppressiven gerichteten Antiserum zeigt. Diese Kreuzreaktivitäten liegen für verschiedene Metaboliten zwischen 0 und 30%, wobei die relative Verteilung der Metaboliten im Blut zudem zeitabhängig ist und starke individuelle Unterschiede zeigt. Die mit dem RIA-Test gewonnenen Daten geben daher weder eine genaue Auskunft über die Menge an Immunsuppressivum, noch lassen sie einen Schluß auf die Gesamtmenge an biologisch aktiven Teilen (Immunsuppressivum + aktive Metaboliten) zu.

Beide beschriebenen Methoden liefern somit Daten, die nur in bedingtem Maß für die klinische Praxis relevant sind.

Der Erfindung liegt die Aufgabe zugrunde, ein Verfahren der eingangs genannten Art zu schaffen, welches ermöglicht, selektiv nur diejenigen Anteile an Immunsuppressiva zu erfassen, die auch biologisch aktiv sind.

Erfindungsgemäß wird diese Aufgabe dadurch gelöst, daß der zu untersuchenden Blutprobe eine definierte Menge an Cyclophilin und eine definierte Menge an markierter, an Cyclophilin bindbarer Substanz zugesetzt wird, worauf dann der an das Cyclophilin gebundene Anteil an markierter Substanz durch Vergleich mit Standards der Pegel von Cyclosporin-A, bzw. der diesem verwandter Immunsuppressiva sowie jener deren Metaboliten gemessen wird. Dadurch werden gerade jene Mengen an biologisch aktiven Anteilen der Immunsuppressiva bestimmt, die zum Zeitpunkt der Ermittlung biologische Aktivität zeigen. Die nach dem erfindungsgemäßen Verfahren ermittelten Werte liegen dabei in allen Fällen gleich hoch oder höher als jene nach der HPLC-Methode ermittelten Werte, jedoch häufig etwas niedriger als die im RIA-Test erhaltenen Werte.

Bei dem eingesetzten Cyclophilin handelt es sich dabei um ein Protein, welches als Bindeprotein für Cyclosporin wirkt. Das Cyclophilin wurde von Handschumacher und Mitarbeitern 1984 in Science 226, Seiten 544 bis 547, beschrieben.

Damit nach dem erfindungsgemäßen Verfahren auch Vollblutproben aufgearbeitet werden können, ohne daß die Ergebnisse durch andere Blutbestandteile verfälscht werden, können vorteilhafterweise in der zu untersuchenden Blutprobe vor der Analyse die Blutzellen hämolysiert und die Serumproteine ausgefällt werden. Dazu kann die zu untersuchende Blutprobe nach Hämolyse der Blutzellen und Ausfällen der Serumproteine zentrifugiert, danach der Überstand eingedampft, anschließend der Rückstand mit einem Lösungsmittel, vorzugsweise Äthanol, aufgenommen und gegebenenfalls nochmals zentrifugiert werden. Dadurch wird eine Lösung erhalten, in welcher praktisch keine, die erfindungsgemäße Reaktion beeinträchtigenden Substanzen mehr enthalten sind. Vorteilhafterweise kann erst nach dieser Vorbehandlung der äthanolische Probenextrakt mit der definierten Menge an Cyclophilin und der definierten Menge an markierter, an Cyclophilin bindbarer Substanz inkubiert werden, worauf dann die nicht an das Cyclophilin gebundene Menge der markierten Substanz aus der Lösung abgetrennt wird, wobei vorzugsweise ein Bindungspuffer hoher Ionenstärke eingesetzt wird. Dadurch wird erreicht, daß nur jener Teil an markierter Substanz der Auswertung zugeführt wird, der tatsächlich an Cyclophilin gebunden ist. Durch den Einsatz des Bindungspuffers hoher Ionenstärke wird eine bessere Bindung des Cyclosporins am Cyclophilin erzielt. Die Abtrennung der nicht gebundenen Anteile an markierter Substanz kann dabei besonders vorteilhaft mittels Aktivkohle durchgeführt werden.

Das erfindungsgemäße Verfahren wird nachstehend anhand eines Beispieles näher erläutert.

Herstellung des Cyclophilin-Extraktes aus menschlichen Zellen

Als Quelle für die Cyclophilin-Extrakte können periphere Blutzellen oder in Suspensionskultur wachsender Tumorzellinien verwendet werden. Die vorliegend verwendeten Zellinien sind die menschlichen T-Zellinien CCRF-CEM und JURKAT sowie die Fibroblastenlinie HeLa. Alle Linien können in RPMI 1640 Medium mit 10% fötalem Kälberserum (FCS) gezüchtet werden. Die Zellen werden bis zu einer Dichte von 1-2 Millionen Zellen pro Milliliter Kulturmedium wachsen gelassen und dann durch Zentrifugation (15 Minuten bei 1500 rpm) geerntet. Sie werden zweimal mit Phosphat-Salz-Puffer, pH 7,2 (10 mmol/l Phosphat, 145 mmol/l NaCl) gewaschen und können bei -20°C gelagert werden.

500 Millionen Zellen werden in 2 ml Cyclophilin-Präparationspuffer (20 mmol/l Tris-HCl, pH 7,5; 1 mmol/l Dithiothreitol (DTT); 0,02% Natriumazid) suspendiert und durch mehrmaliges Einfrieren und Auftauen lysiert. Das Lysat wird entweder 2 min in einer Eppendorfzentrifuge oder 10 min bei 4000 rpm in einer Beckmann J-6B Zentrifuge zentrifugiert. Die Überstände werden in ein Glasröhrchen transferiert, das Zentrifugat in 1ml Präparationspuffer resuspendiert und nochmals in der angegebenen Weise zentrifugiert. Die Überstände werden vereinigt, in 0,5ml Aliquote portioniert und bei -20°C gelagert. Die so gewonnene Cyclophilin-Menge reicht für etwa 100 Cyclosporin Bestimmungen.

Probenbereitung:

Extraktion von Cyclosporin-A (CsA) aus Vollblut

Proben zu je 1 ml Vollblut werden in Glasspitzröhrchen unter Schütteln mit je 2ml Acetonitril versetzt, was die Hämolyse der Blutzellen sowie die Ausfällung von Serumproteinen und zellulären Bestandteilen bewirkt. Die Röhrchen werden anschließend 5 min bei 3500 rpm in einer Beckmann J-6B Zentrifuge zentrifugiert, 2ml des Überstandes werden entnommen, in neue Spitzröhrchen transferiert und im Wasserbad bei 37°C im Stickstoffstrom eingedampft, in 667 µl 60% Äthanol aufgenommen und eine Minute gschüttelt. Dadurch wird die nahezu vollständige Auflösung des in den Proben enthaltenen CsA gewährleistet. Die Röhrchen werden noch einmal 5 min 3500 rpm zentrifugiert und die Überstände in Glasröhrchen oder Eppendorfgefäßen bei -20°C gelagert. Bei dieser Vorgangsweise entspricht 1 ml gelöster Extrakt 1 ml Vollblut. Als Kontrollen dienen Proben von cyclosporinfreiem Blut, denen definierte Mengen an Cyclosporin-A zugesetzt werden. (Konzentrationsbereich 200 - 2000 ng/ml).

Der Bindungsassay

Das Prinzip des Assays besteht ähnlich wie beim RIA darin, eine genau definierte Menge an radioaktiv markiertem Cyclosporin-A (3H)CsA an Cyclophilin zu binden und die Verdrängung des radioaktiven (3H)CsA durch das in der extrahierten Probe vorhandene nicht radioaktive Cyclosporin-A bzw. dessen biologisch aktive Metaboliten zu messen. Als Bezugssubstanz zur Erstellung einer Standardkurve dient unmarkiertes CsA. Folgende Reagenzien werden benötigt:
Bindungspuffer: 50 mmol/l Tris-HCl, pH 7,5; 10% Fötales Kälberserum (FCS); 2 mmol/l Dithiothreitol (DTT); 0,04% Natriumazid. Es kann auch ein Bindungspuffer hoher Ionenstärke eingesetzt werden, der folgende Zusammensetzung aufweist:
50 mmol/l Tris-HCl, pH 7,5; 1 mol/l Ammoniumsulfat, 5% FCS, 2mmol/l DTT, 0,04% Natriumazid.
(3H)CsA (Sandoz): 7,4 kBq/ml (0,2 µCi/ml) in 10% Äthanol (Originallösung 1:10 mit destilliertem Wasser verdünnt).
Blutextrakte von Patienten und Kontrollpersonen in 60% Äthanol gelöst.
CsA-Standardlösung (Sandoz): 4µg/ml (Originallösung 1:10 mit KontrollblutExtrakt verdünnt).
Zur Herstellung eines 1ml Inkubationsansatzes werden die folgenden Mengen in ein Glasröhrchen pipettiert:
250 µl Blutextrakt
200 µl (3H)CsA
550 µl Bindepuffer, der 50 µl Zellextrakt/ml enthält.
Zur Erstellung der Standardkurve werden verschiedene Mengen von CsA in Kontrollblutextrakten gelöst und in derselben Weise wie die Patientenblutextrakte im Assay eingesetzt. Die Konzentration der Standards sollten zwischen 200 und 2000 ng/ml Extrakt liegen. Zur Bestimmung der Menge des an Cyclophilin gebundenen (3H)CsA muß das nicht gebundene quantitativ abgetrennt werden. Dazu wird das nicht gebundene Cyclosporin durch Adsorption an Aktivkohle wie folgt ausgefällt:
2 g Aktivkohle (Merck No. 2186) werden in 100 ml Säulenpuffer, der 1% fötales Kälberserum (FCS) enthält suspendiert und über Nacht im Kühlschrank gerührt. Durch diese Behandlung wird die Bindung von Proteinen an die Aktivkohle erschwert, während Cyclosporin noch bereitwillig adsorbiert wird.

In Glasröhrchen (75 x 12mm) werden Bindungsansätze zu je 0,5 ml in der angegebenen Weise bereitet und nach 10 min Inkubation bei Raumtemperatur in ein Eisbad transferiert, dort 15 min belassen und dann mit je 0,25 ml eisgekühlter Aktivkohlensuspension versetzt. Unmittelbar nach der Aktivkohlenzugabe werden die Proben kurz geschüttelt, noch weitere 10 min im Eisbad belassen und dann 15 min bei 3500 rpm zentrifugiert. Die Überstände werden in Szintillationsfläschchen dekantiert, mit 7 ml Szintillations- flüssigkeit versetzt und die Radioaktivitäten gemessen.

Ergebnisse

Die mit der beschriebenen Methode gewonnenen Ergebnisse liefern folgendes Bild:
Die Werte der Cyclosporin-Spiegel liegen in allen Fällen höher als die mit HPLC bestimmten und häufig etwas niedriger als die im RIA ermittelten. Dabei scheint der Zeitpunkt, der zwischen CsA-Einnahme und Blutabnahme liegt, wesentlich zu sein (Fig.1). Zu Beginn, d.h. in den ersten 4 Stunden, liefern HPLC und Cyclophilin-Assay nahezu identische Werte, danach sinken die HPLC-Werte wesentlich schneller ab, während die Cyclophilin-Werte sich kontinuierlich denen des RIA annähern und nach 12 Stunden von derselben Größenordnung sind.
Betrachtet man individuelle Verläufe über mehrere Tage hinweg, bei denen die Blutabnahme meist 10 Stunden nach CsA Einnahme erfolgte - die Meß- punkte also dem Endbereich des Tagesprofils entsprechen - so liegen die Cyclophilin-Werte deutlich höher als die HPLC Werte, manchmal auch höher als die des RIA (Fig.2) . Die Methode der Cyclosporinbestimmung durch Bindung an Cyclophilin liefert also Ergebnisse, die sich von den mit den beiden bekannten und derzeit ausschließlich verwendeten Ergebnissen deutlich unterscheiden. Dies rührt offenbar daher, daß Cyclophilin außer CsA auch einen Teil der Metaboliten zu binden vermag, und zwar vor allem jene mit immunsuppresiver Wirkung.

Es steht somit zum ersten Mal eine Methode zur Verfügung, biologisch relevante Cyclosporin-Spiegel im Blut zu bestimmen, da mit diesem Assay nur diejenigen Cyclosporine erfaßt werden, die auch immunsuppressive Wirkung aufweisen.

**Patentansprüche**

1. Verfahren zur Bestimmung des aktuellen Pegels von Cyclosporin-A bzw. des diesem verwandter Immunsuppressiva sowie jener deren biologisch aktiver Metaboliten im Blut, dadurch gekennzeichnet, daß der zu untersuchenden Blutprobe eine definierte Menge an Cyclophilin und eine definierte Menge an markierter an Cyclophilin bindbarer Substanz zugesetzt wird, worauf dann der an das Cyclophilin gebundene Anteil an markierter Substanz durch Vergleich mit Standards der Pegel von Cyclosporin-A, bzw. der diesem verwandter Immunsuppressiva sowie jener deren Metaboliten gemessen wird.

2. Verfahren nach Anspruch 1, dadurch gekennzeichnet, daß in der zu untersuchenden Blutprobe vor der Analyse die Blutzellen hämolysiert und die Serumproteine ausgefällt werden.

3. Verfahren nach Anspruch 2, dadurch gekennzeichnet, daß die zu untersuchende Blutprobe nach Hämoylse der Blutzellen und Ausfällen der Serumproteine zentrifugiert, danach der Überstand eingedampft, anschließend der Rückstand mit einem Lösungsmittel, vorzugsweise Äthanol, insbesondere 60%-igem Äthanol, aufgenommen und gegebenenfalls nochmals zentrifugiert wird.

4. Verfahren nach Anspruch 3, dadurch gekennzeichnet, daß der äthanolische Probenextrakt mit der definierten Menge an Cyclophilin und der definierten Menge an markierter an Cyclophilin bindbarer Substanz inkubiert wird, worauf dann die nicht an das Cyclophilin gebundene Menge an markierter Substanz aus der Lösung abgetrennt wird, wobei vorzugsweise ein Bindungspuffer hoher Ionenstärke eingesetzt wird.

5. Verfahren nach Anspruch 4, dadurch gekennzeichnet, daß die Abtrennung der nicht gebundenen Anteile der markierten Substanz mittels Aktivkohle durchgeführt wird.